# EUROPEAN PATENT SPECIFICATION

(11) **EP 4 204 129 B1**
(45) Date of publication and mention of the grant of the patent: **05.11.2025**
(21) Application number: 21758629.6
(22) Date of filing: 04.08.2021
(51) Int. Cl.: B01D 63/02, B01D 65/00, A61M 1/16

(54) **METHOD FOR VENTING A HOLLOW FIBER MEMBRANE FILTER AND HOLLOW FIBER MEMBRANE FILTER DEVICE**
VERFAHREN ZUM ENTLÜFTEN EINES HOHLFASERMEMBRANFILTERS UND HOHLFASERMEMBRANFILTERVORRICHTUNG
PROCÉDÉ DE PURGE D'UN FILTRE À MEMBRANE À FIBRES CREUSES ET DISPOSITIF DE FILTRE À MEMBRANE À FIBRES CREUSES

(30) Priority: 31.08.2020 EP 20193648
(43) Date of publication of application: 05.07.2023
(73) Proprietor: BRITA SE, 65232 Taunusstein (DE)
(72) Inventor: SOBEK, Nicoletta, 65232 Taunusstein (DE)
(86) International application number: PCT/EP2021/071721
(87) International publication number: WO 2022/043011

(56) References cited:
- WO-A1-2015/014934
- WO-A2-2008/026079
- US-A1- 2012 074 063
- US-B2- 10 421 042
- US-B2- 10 500 328

## Description

### Technical Field

The invention relates to a method for venting a hollow fiber membrane filter before first use of the filter. The invention also relates to a hollow fiber membrane filter device that allows for performing this method for venting before first use of this hollow fiber membrane filter.

### Background of the invention

For many different fields of application, a hollow fiber membrane filter can be used to remove unwanted particles or constituents from a liquid stream. Hollow fiber membrane filters comprising polymer or ceramic membranes, **e.g.** silica-based membranes, hydrophilic cellulose acetate membranes or hydrophobic polyether sulfone membranes can be used for the filtration of drinking water, for water treatment or for water reuse applications. Hollow fiber membrane devices are also used in biological or medical applications, **e.g. as** substrates for bioreactor systems or as part of dialysis devices.

WO 2008/026079 A1 discloses a liquid filtration apparatus that comprises front and back sheets that are liquid-impermeable and joined to each other along seams to form first and second reservoirs. An intermediate seam joins the front and rear sheets directly together along a pair of distal segments extending inwardly from respective opposing side seams. Between the distal segments, a central segment of the intermediate seam joins the front and back sheets to respective front and rear sides of a filter module, around a filter inlet provided therein. The filter module comprises a housing defining a filter chamber that is generally cylindrical in shape, and that has one outlet located at each end. The inlet is located along a sidewall portion of the housing, intermediate the opposed outlets, and is oriented to face upwards in the apparatus to facilitate receiving liquid into the inlet from the first reservoir. A fin is sandwiched between the front and back sheets along the intermediate seam of the apparatus. The fin has a front wall and back wall that are spaced apart from each other at an intermediate location along the length of the housing between the opposing ends to define the filter inlet. The front and back walls of the fin provide respective front and back surfaces to which the front and back sheets are respectively bonded along the central segment of the intermediate seam. Untreated liquid in the first reservoir is prevented from directly flowing into the second reservoir by the distal segments of the intermediate seam, and can enter the second reservoir only by passing through the filter module via the filter inlet and outlet. Suspending the apparatus in a vertical position can allow gravity to draw the untreated liquid from the first reservoir into the inlet of the filter module.

For many hollow fiber membrane filters there is a need to remove air from the hollow fiber material within the hollow fiber membrane filter, and to fill the hollow fiber material with a liquid in order to prepare the hollow fiber material before the first use of the hollow fiber membrane filter. Otherwise, a first amount of liquid that is forced through the hollow fiber membrane filter will not be fully treated by the hollow fiber material, and the efficiency of the hollow fiber material will be reduced during subsequent filtering treatments of a liquid streaming through the hollow fiber material.

For water treatment applications, the hollow fiber membrane filter must be inserted into a filtering device comprising two compartments arranged one above the other and separated by the hollow fiber membrane filter. After filling the upper compartment with water, the weight of the water causes the water to flow through the hollow fiber membrane filter into the lower compartment, resulting in a filtering treatment of the water. However, also hollow fiber membrane filters that are inserted into such filtering devices must be vented after removing the hollow fiber membrane filter from a transport packaging and before first use of the hollow fiber membrane filter.

It is known from practice to arrange the hollow fiber membrane filter below a funnel and, after adding a generous amount of water into the funnel, to wait until the water has flowed from the funnel through the hollow fiber membrane filter, thereby venting the hollow fiber material. However, a funnel is required and it usually takes some time for the water to flow through the hollow fiber membrane filter. It is also possible to insert the hollow fiber membrane filter into the filtering device and to fill the upper compartment with water. After all the water has flown from the upper compartment through the hollow fiber membrane filter into the lower compartment, the water is removed from the lower compartment, but not used for human consumption, as the filtering efficiency of the hollow fiber membrane filter only increases gradually during the venting process. This step can be performed repeatedly until the venting of the hollow fiber membrane filter is completed. Even though such a venting procedure does not require an additional funnel, it takes some time for the repeated filling of the upper compartment, the waiting for the water flow through the hollow fiber membrane filter, and for the emptying of the lower compartment during the venting process.

Thus, there is a need for a method for venting the hollow fiber membrane filter that can be easily and quickly performed.

### Summary of the invention

The present invention relates to a method as defined in claim 1 for venting a hollow fiber membrane filter that is provided within a transport packaging. During a pouch filling step a quantity of a liquid is filled at the first end into a cavity formed by a flexible and liquid-tight open pouch that is sealed liquid-tight along a circumferential surface of a housing of the hollow fiber membrane filter at the first front side of the housing of the hollow fiber membrane filter, and in that during a venting step the quantity of liquid in the cavity is forced through the hollow fiber membrane filter to exit at the second front side of the casing of the hollow fiber membrane filter. Thus, the pouch provides for a cavity that can be filled with the liquid that is required for venting the hollow fiber membrane filter during the venting step. Thus, there is no need for an additional device like **e.g.** a funnel that is usually required for performing the venting step according to prior art.

It is possible to exert pressure on the cavity to force the liquid quickly through the hollow fiber membrane filter. Thus, the time that is required for the amount of liquid to pass through the hollow fiber membrane filter can be significantly reduced by this method.

It is possible to provide for a large pouch that is large enough to be filled with an amount of liquid that is enough to complete the venting process after a single pass of the liquid from the closed cavity through the hollow fiber membrane filter. However, it is also possible to repeat the pouch filling step and the venting step once or several times until enough liquid is forced through the hollow fiber membrane filter for completely venting the hollow fiber membrane filter.

According to the invention, during the pouch filling step after the filling of the liquid into the cavity through a first opening at a first end of the pouch, the first end of the pouch is closed in order to enclose the quantity of the liquid in the pouch at the first front side of the housing of the hollow fiber membrane filter. By closing the cavity, it is very easy to exert pressure to the cavity by compressing cavity section of the flexible pouch, i.e. by reducing the size of the closed cavity, resulting in an increase of pressure that forces the liquid through the hollow fiber membrane filter. The first end of the pouch can be closed by manually squeezing the pouch material at or near the first end. It is also possible to fold the pouch once or several times near the opening at the first end in order to close the opening or to shut-off the opening from the cavity.

In yet another embodiment of the invention, during the pouch filling step and after the filling of the liquid into the cavity the first opening of the pouch is closed by several winding movements of the first end of the pouch around a winding axis. By winding the first end of the transport packaging around a winding axis, the first opening at the first end can be tightly sealed without much efforts and without a greater risk of making mistakes in the process. The winding axis should be approx. parallel to the surface of the first front side of the housing of the hollow filter membrane filter. However, there is no need for the winding axis to be parallel to this surface, as it is only required to provide for a liquid-tight sealing of the first end of the transport packaging. The distance between the opening at the first end of the pouch and the first front side of the housing of the hollow filter membrane filter is preferably larger than required by the closed cavity in order to facilitate the handling of the pouch and to enable several winding movements of the first end with the cavity filled by the liquid. If the filled cavity extends for e.g. 5 centimeters, the distance between the opening at the first end and the first front side of the housing of the hollow filter membrane filter can be e.g. 10 centimeters or more, allowing for at least 5 centimeters to be wound around a winding axis that is parallel to the first front side which is considered enough to provide for a liquid-tight sealing. However, it is also possible and possibly further facilitates the handling if the distance between the opening at the first end and the filling level of the cavity is more than 5 centimeters, e.g. 10 centimeters, 15 centimeters or any other distance considered large enough.

The winding movements of the first end of the pouch can be performed manually without any supporting element. However, in order to further facilitate the winding movements, the winding movements can be performed around a winding axis element like e.g. a stick or a rod that is manually arranged along the winding axis. The winding axis element can be a pen, a cooking spoon or something alike that is available to the user that performs the venting of the hollow filter membrane filter. It is also possible to attach a winding axis element like a stick e.g. to the housing of the hollow fiber membrane filter or to a transport packaging, and to provide the winding axis element together with the hollow fiber membrane filter for use during the venting process.

According to the invention, the size of the cavity formed by the closed first end of the pouch is reduced during the venting step, thereby forcing the liquid through the hollow fiber membrane filter. By reducing the size of the cavity, pressure is exerted to the liquid that has been filled into the cavity, and the liquid is forced by pressure through the hollow filter membrane filter. The larger the pressure, the faster the venting process can be performed and completed, and the quicker the hollow fiber membrane filter is ready for mounting the hollow fiber membrane filter into the filtering device.

In accordance with a particularly advantageous embodiment of the invention, the size of the cavity is reduced by manually compressing the flexible pouch. This can be performed **e.g. by** adding further winding movements at the first end of the pouch, thus reducing the size of the cavity by continuously moving the winded and liquid-tight sealed first end of the pouch towards the first front side of the housing of the hollow filter membrane filter. It is also possible to press together the side walls of the pouch within the cavity section. In case that the diameter of the pouch within the cavity section is approx. 10 centimeters or less, the cavity section can be gripped and compressed with one hand. It is also possible and advantageous for large diameter cavity sections that the cavity section is placed on a supporting surface and then pressed together with one hand, which is placed on the cavity section from above.

In accordance with an aspect of the invention, after venting the hollow fiber membrane filter, at least one part of the pouch that protrudes over the housing of the hollow fiber membrane filter housing is separated and removed. It is also possible to completely remove the pouch from the housing of the hollow fiber membrane filter. The liquid-tight sealing of the pouch to the circumferential surface of the housing can be performed by **e.g.** gluing or friction-welding the pouch to the surface in such a manner that it is possible to manually remove the pouch from the surface and thus from the housing after performing the venting step.

According to an embodiment of the invention that is considered very advantageous, a transport packaging of the housing of the hollow fiber membrane filter can be adapted and used for supporting and performing the required venting of the hollow fiber membrane filter before the first use. Thus, during a transport packaging preparation step a flexible and liquid-tight transport packaging, that is sealed liquid-tight along a circumferential surface of a housing of the hollow fiber membrane filter, is opened at a first end and opened at a second end providing first and second openings of the transport packaging with access to corresponding first and second openings of the hollow fiber membrane filter at the opposite front sides of the housing of the hollow fiber membrane filter. The part of the transport packaging that protrudes the first front side of the housing with the first opening of the hollow fiber membrane filter forms the pouch. The transport packaging can be designed and adapted to provide for a pouch at the first front side of the housing that allows for all aspects during the venting of the hollow fiber membrane filter described above. However, no additional material for a separate pouch is required, and no additional manufacturing step for manufacturing the pouch and fastening the pouch to the housing before inserting the housing into a transport packaging is required. Thus, the manufacturing costs can be reduced. Furthermore, due to the reduced material requirements the method can be performed in an ecologically worthwhile and environmentally friendly manner.

According to yet another embodiment of the invention, during a transport packaging removal step, at least one part of the transport packaging with the first end that protrudes over the housing of the hollow fiber membrane filter and at least one part of the transport packaging with the second end is separated from the housing and removed. If all parts of the transport packaging are removed that prior to the removal protrude over the housing of the hollow fiber membrane filter, there will be no interference of these parts of the transport packaging during the mounting of the hollow fiber membrane filter into the filtering device or during subsequent use of the hollow fiber membrane filter for filtering a liquid. In order to facilitate the removal of such parts of the transport packaging it is possible that the areas of the transport packaging to be separated are connected to the remaining areas of the transport packaging via suitable perforations. It is also possible to attach the transport packaging to the housing of the hollow fiber membrane filter in such a manner that allows for easy removal of the complete transport packaging from the housing after performing the venting of the hollow fiber membrane filter. The invention also relates to a hollow fiber membrane filter device with a housing comprising a hollow fiber membrane filter.

Usually, such hollow fiber membrane filters are enclosed within a flexible transport packaging made from a plastic foil or from an impregnated and water-repellant paper material in order to avoid unwanted pollution or contamination of the hollow fiber membrane filter after manufacture and before use within a filtering device, **e.g.** during times of storage at a distributor or during times of transport from the manufacturer to the distributor up to the final customer.

Just before mounting the hollow fiber membrane filter into a filtering device, the hollow fiber membrane filter is removed from the transport packaging and must be vented in order to ensure proper operation of the hollow fiber membrane filter during a filtering process.

It is considered an object of the present invention to provide for a hollow fiber membrane filter that facilitates the required preparations before mounting the hollow fiber membrane filter into a filtering device. The invention provides a hollow fiber membrane filter device as defined in claim 7.

According to an advantageous aspect of the invention, a flexible and liquid-tight open pouch is sealed liquid-tight along a circumferential surface of a housing of the hollow fiber membrane filter at a first front side of the housing of the hollow fiber membrane filter. Such a pouch can be easily mounted and sealed to the housing of the hollow fiber membrane filter, e.g. by gluing, by friction-welding or by heat shrinking. The weight of the pouch does not significantly contribute to the weight of the hollow fiber membrane filter device. However, by adding a pouch to the housing of the hollow fiber membrane filter, the venting of the hollow fiber membrane filter before its first use can be considerably facilitated and accelerated. The pouch can be manufactured from a flexible and liquid-tight foil. The design of the pouch can be adapted to provide for a cavity section that is large enough to hold an amount of liquid that is enough for fully venting the hollow fiber membrane filter.

The shape of the pouch is adapted to be sealed in a liquid-tight manner onto the circumferential surface of the housing of the hollow fiber membrane filter. For most applications, a cross-section of the housing will be circular or oval. The cross-section of the pouch within a sealing section of the pouch is preferably adapted to be similar to the cross-section of the housing. The pouch can be tube-shaped. However, it is also possible to provide for a pouch with a conical or bellied shape.

According to yet another advantageous aspect of the invention, the transport packaging is made of a flexible and liquid-tight transport packaging material, and the transport packaging is sealed liquid-tight along a circumferential surface of the housing of the hollow fiber membrane filter with a first end of the transport packaging covering a first front side of the housing and with a second end of the transport packaging covering a second front side of the housing, whereby the pouch is formed by a cavity section between the first end part of the transport packaging and the first front side of the housing of the hollow fiber membrane filter. After opening the first end and the second end of the transport packaging, a user can fill a liquid into the opening of the first end of the transport packaging that flows through the hollow fiber membrane filter and discharges from the transport packaging through the second opening at the other side of the housing of the hollow fiber membrane filter. Thus, the transport packaging can be used to provide for the pouch and to support the venting process and to allow a user to perform the venting process without need for additional tools like e.g. a funnel for venting the hollow fiber material within the hollow fiber membrane filter.

According to an embodiment of the invention, a cross-section of the transport packaging is adapted to a cross-section of the housing along the circumferential surface of the housing with the liquid-tight sealing. Thus, the transport packaging can be shrink-fitted or welded onto the lateral surface of the housing without significant deformation of the shape of the transport packaging at or around the liquid-tight sealing. Adapting the cross-section of the transport packaging to the corresponding cross-section of the housing along the liquid-tight sealing facilitates the liquid-tight fastening of the transport packaging to the housing. Furthermore, the size of the transport packaging can be minimized.

In accordance with an embodiment of the invention the housing of the hollow fiber membrane filter is tube-shaped and the transport packaging is tube-shaped. A tube-shaped housing provides for a circular cross-section of the first front side and the second front side of the housing. The hollow fibers can run in a straight line from the first front side to the second front side. Such a design allows for a high performant filtering device with a very efficient use of the hollow fiber material that is arranged inside of the housing. A tube-shaped transport packaging can be easily and cost-effectively manufactured from a film tube. After inserting the housing in a section of such a film tube, the first end and the second end of the film tube are closed, resulting in sealing the housing within the film tube between the two closed ends. The first end and the second end of the film tube are on opposite sides of the housing and both first and second end are arranged at a distance to the respective first front side and second front side of the housing. Thus, each of the first and second end of the film tube can be pressed together to a flat line and closed e.g. by ultrasonic welding or by using an adhesive.

In order to facilitate the required preparations of the transport packaging before performing the venting process as described above, it is considered advantageous that a first perforation is arranged at the first end of the transport packaging that allows for removal of a first end part of the transport packaging at a distance to the housing of the hollow fiber membrane filter, resulting in a cavity formed by a cavity section of the first end of the transport packaging between the removed first end part of the transport packaging and the first front side of the housing of the hollow fiber membrane filter. The first perforation enables a user to remove a first end part of the transport packaging and to provide for a first opening of the transport packaging through which the liquid can be filled into the cavity section of the transport packaging that is used for the venting process. The first perforation can be designed to run along a circumference of the transport packaging, preferable along a circumference of a tube-shaped transport packaging, resulting in a large first opening at a distance to the first front side of the housing of the hollow fiber membrane filter inside the transport packaging.

According to an embodiment of the invention, the first end of the transport packaging also comprises a closure section between the cavity section and the first end part of the transport packaging that allows for closing the cavity section after removal of the first end part of the transport packaging. Thus, the first perforation is preferably arranged at a distance to the first front side of the housing of the hollow fiber membrane filter that is large enough to allow for a cavity section and a closure section between the first perforation at the first end of the transport packaging and the first front side of the housing. Depending on the volume of the hollow fiber membrane filter that requires the venting process, the volume of the cavity section is adapted to the volume of the hollow fiber membrane filter and can be **e.g.** the same volume or double the volume of the hollow fiber membrane filter. The size of the closure section is preferably at least some centimeters in order to allow for an easy handling during a closing step that is required for closing the first opening of the transport packaging after filling the cavity with the liquid. For many shapes of the transport packaging, the length of the closure section, **i.e.** the distance between the first opening and the cavity section should be at least some centimeters, preferably more than 5 or 10 centimeters.

The size, and more specific the length of the closure section that is measured as the distance between the cavity section and the first opening of the transport packaging, should be preferably large enough to allow for some winding movements with which an end section of the transport packaging is wound together with the open end, thereby closing the first opening in a liquid-tight manner without the need for additional tools or elements, but only with some winding movements of the hands of a user. However, if deemed helpful or for further facilitating the venting process and in particular the closure of the cavity with winding up the first end of the transport packaging, a separate stick or rod can be provided that is removably attached to the outside or to the inside of the transport packaging. Such a stick or rod can be removed by a user and arranged at the first opening of the first end of the transport packaging in a manner that supports the winding movements and in particular the process of rolling up or wrapping up the first end of the transport packaging to obtain a liquid-tight closure of the transport packaging and, more specific, the cavity that has been filled with the liquid to perform the venting process of the hollow fiber membrane filter. The length of such a stick or rod should be larger than the diameter of the first opening of the transport packaging after pressing together the transport packaging at the first opening, i.e. after flattening the transport packaging near and at the first opening.

In order to reduce the handling effort required to open the transport packaging at a second end which is located opposite the first end on the other side of the housing, optionally a second perforation is arranged at the second end of the transport packaging that allows for removal of a second end part of the transport packaging. The removable second end part can be designed as a slit or as an opening of rectangular or circular shape at the second end of the transport packaging. It is also possible to provide for a second perforation that is similar to the first perforation and runs along a circumference of the transport packaging at the second end of the transport packaging. Thus, the second opening can be of similar shape like the first opening, resulting in a large diameter second opening with a cross-section that is similar or identical to the cross-section of the transport packaging near or at the second end of the transport packaging. In case of a tube-shaped transport packaging, the second opening can be of circular shape with a cross-section identical to the cross-section of the tube-shaped transport packaging.

According to another embodiment of the invention, a third perforation is arranged at the first end of the transport packaging next to the fluid-tight sealing of the transport packaging along the circumferential surface of the housing of the hollow fiber membrane filter. Furthermore, according to yet another embodiment of the invention, a fourth perforation is arranged at the second end of the transport packaging next to the fluid-tight sealing of the transport packaging along the circumferential surface of the housing of the hollow fiber membrane filter. By providing such third and fourth perforations next to the fluid-tight sealing of the transport packaging along the circumferential surface of the housing of the hollow fiber membrane filter, at least one part of the transport packaging that protrudes over the housing of the hollow fiber membrane filter can be easily separated from the housing and removed. Preferably, both parts of the transport packaging that protrude over the housing are removed before a filtering process begins. However, it is possible to remove **e.g.** the first end of the transport packaging comprising the closure section and the cavity section are removed before the hollow fiber membrane filter is inserted into a filter seating within the filtering device. After inserting the hollow fiber membrane filter into the filtering device by only touching the second end of the transport packaging, the second end of the transport packaging can also be removed by pulling the second end from the housing of the hollow fiber membrane filter, which severs the fourth perforation and separates the second end of the transport packaging from the housing.

Instead of some or all perforations, one or more of the first to fourth perforation can be replaced by indications or separation lines that are imprinted onto the transport packaging. Thus, no perforation is required within the transport packaging. By adding indications or separation lines that are imprinted onto the transport packaging, this will provide for an explicit teaching of how to open and to remove the transport packaging during and after venting the hollow fiber membrane filter.

Optionally, the transport packaging is made of or comprises a transport packaging material that is a biodegradable or recycled material.

### Brief description of the drawings

The present invention will be more fully understood, and further features will become apparent, when reference is made to the following detailed description and the accompanying drawings. The drawings are merely representative and are not intended to limit the scope of the claims. Like parts depicted in the drawings are referred to by the same reference numerals.
Figure 1 illustrates a perspective view of a hollow fiber membrane filter with a tube-shaped housing arranged inside of a transport packaging, whereby for the sake of clarity the transport packaging is made of a transparent transport packaging material,
Figure 2 illustrates a cross-sectional view along the line II-II in figure 1 of the hollow fiber membrane filter with the transport packaging shown in Figure **1****,**
Figure 3 illustrates a perspective view of the hollow fiber membrane filter of figures 1 and 2 after filling a cavity section of the transport packaging with a liquid,
Figure 4 illustrates a perspective view of the hollow fiber membrane filter similar to figure 3 after closing the cavity section of the transport packaging that comprises the liquid,
Figure 5 illustrates a perspective view of the hollow fiber membrane filter similar to figures 3 and 4 during the compression of the cavity section of the transport packaging which forces the liquid through the hollow fiber material of the hollow fiber membrane filter,
Figure 6 illustrates a perspective view of the hollow fiber membrane filter after removal of two end parts of the transport packaging that otherwise protrude over the housing of the hollow fiber membrane filter,
Figure 7 illustrates a perspective view of another embodiment of the hollow fiber membrane filter with a transport packaging that can be removed differently to the embodiment shown in Figure **6****,** and
Figure 8 illustrates a perspective view of yet another embodiment of the hollow fiber membrane filter with a pouch that is sealed liquid-tight to the housing of the hollow fiber membrane filter.

### Detailed description of the invention

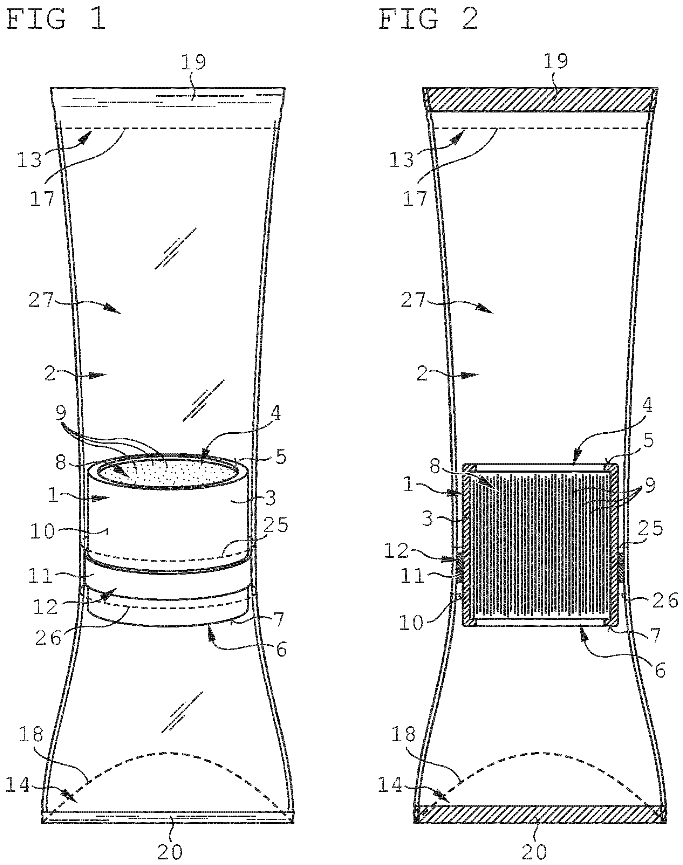

By way of example, figure 1 and figure 2 illustrate a perspective view and a cross-sectional view of a hollow fiber membrane filter 1 inside of a flexible transport packaging **2.** For the purpose of demonstrating some aspects of the invention, the transport packaging 2 is illustrated as being made of a transparent transport packaging material. However, the transport packaging 2 can be made of any suitable transport packaging material like **e.g.** a flexible plastic film or an impregnated paper material which is impermeable to water for at least a few minutes. Preferably, the transport packaging material is a biodegradable or recycled material. The hollow fiber membrane filter 1 comprises a tube-shaped housing 3 with a first opening 4 at a first front side 5 of the housing 3 and with a second opening 6 at a second front side 7 of the housing that is opposite to the first opening 4 and the first front side 5 of the housing 3. Thus, the shape of the housing 3 is similar to a cylindrical shell. Inside of the housing 3 there is a hollow fiber material 8 with a large number of hollow fibers 9 that run from the first front side 5 to the second front side 7 of the housing 3 and that are arranged parallel to each other. The hollow fiber membrane filter 1 can be used to remove unwanted particles or constituents from a liquid stream that passes through the hollow fiber membrane filter 1 by entering through the first front side 5, by streaming through the hollow fiber material 8 and by emitting through the second front side 7 of the housing 3.

The hollow fiber membrane filter 1 is arranged inside the transport packaging 2 made from a tube film with a cross-section that is adapted to the cross-section of the hollow fiber membrane filter 1. The hollow fiber membrane filter 1 is fixed to the transport packaging 2 along a circumferential surface 10 of the housing 3 by shrinking the transport packaging 2 onto a ring of adhesive 11 that provides for a liquid-tight sealing 12 along a circumferential line. It is also possible to fasten the transport packaging 2 with the housing 3 of the hollow fiber membrane filter 1 by means of e.g. ultrasonic welding or gluing with an appropriate adhesive that provides for a liquid-tight sealing 12.

A first end 13 of the tube-shaped transport packaging 2 has been sealed by pressing the first end 13 flat and then closing it by e.g. ultrasonic welding. A second end 14 opposite to the first end 13 and at the other side of the hollow fiber membrane filter 1 has also been sealed by pressing the second end 14 flat and then closing it be e.g. ultrasonic welding. Thus, the transport packaging 2 made from a tube film provides for a sealed wrapping of the hollow fiber membrane filter 1 during transport and storage of the hollow fiber membrane filter 1 until beginning with the venting process and subsequent use of the hollow fiber membrane filter 1 with a filtering device.

In order to perform the required venting of the hollow fiber material before first use of the hollow fiber membrane filter 1, during a transport packaging preparation step, the flexible and liquid-tight transport packaging 2 is opened at a first end 13 and opened at a second end 14 providing first and second openings 15, 16 of the transport packaging 2 with access to corresponding first and second openings 4, 6 of the hollow fiber membrane filter 1 at the opposite front sides 5, 7 of the housing 3 of the hollow fiber membrane filter 1. The manual opening of the first end 13 is supported and simplified by a first perforation 17 that runs along a circumferential line of the tube-shaped transport packaging 2 next to the first end 13 of the transport packaging 2. In the same manner the manual opening of the second end 14 is supported and simplified by a second perforation 18 that runs along a curved line next to the second end 14 of the transport packaging 2. By pulling at a first end part 19 of the transport packaging 2, the first perforation 17 is severed and the first end part 19 with the closure of the tube-shaped transport packaging 2 is removed from the rest of the transport packaging 2, resulting in the first opening 15 at the first end 13 of the transport packaging 2. Similarly, by pulling at a second end part 21 of the transport packaging 2, the second perforation 18 is severed and the second end part 20 with the corresponding closure of the tube-shaped transport packaging 2 is also removed from the rest of the transport packaging 2, resulting in a second opening 16 opposite to the first opening 15. The part of the transport packaging 2 between the first end 13 and the liquid-tight sealing 12 forms a pouch 27. After removing the first end part 19 from the transport packaging 2, the inside of the pouch 27 is accessible through the first opening 15 at the first end 13 of the transport packaging 2.

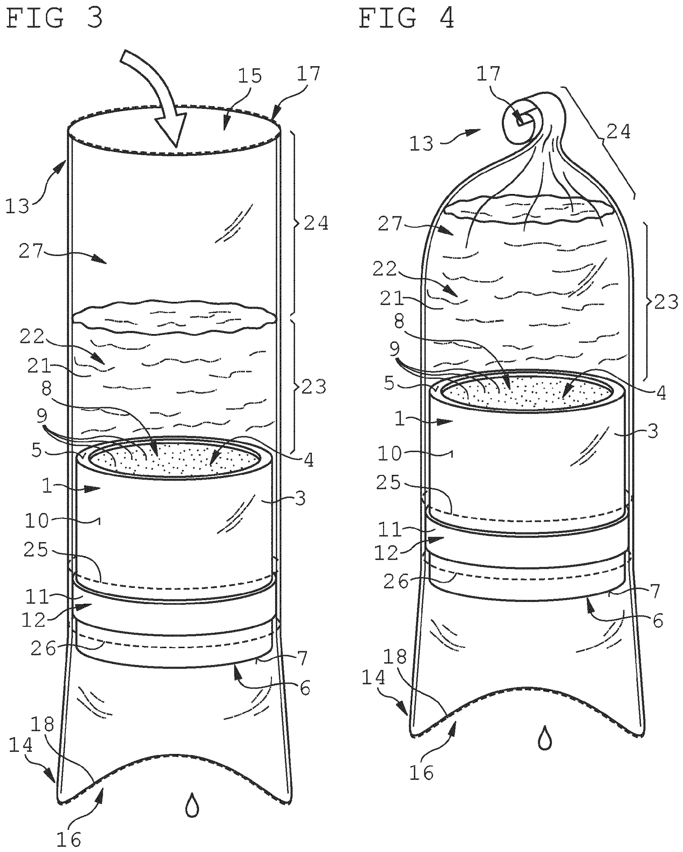

During a following pouch filling step, the transport packaging 2 is arranged with the first opening 15 facing up and the second opening 16 facing down. A quantity of a liquid 21 is filled at the first end 13 through the first opening 20 into a cavity 22 formed by the transport packaging 2 next to the first front side 5 of the housing 3 of the hollow fiber membrane filter 1. Figure 3 shows the cavity 22 within the transport packaging 2 that is filled with the liquid 21, which can be e.g. tap water. The cavity 22 is formed by a cavity section 23 of the transport packaging 2. Due to the gravitational force the liquid 21 slowly soaks through the hollow fiber membrane filter 1 and some droplets exit the housing 3 of the hollow fiber membrane filter 1 at the second front side 7 facing downwards.

Between the first opening 15 facing up and the cavity section 23 below, there is a closure section 24 which can be used for closing the pouch 27, i.e. the cavity 22 after filling the cavity 22 with the liquid 21. The size of the closure section 24, i.e. the distance between the cavity section 23 and the first opening 15 is large enough to allow for some manual winding movements of the first end 13 of the transport packaging 2, resulting in wrapping up at least part of the closure section 24 of the transport packaging 2 beginning with the first end 13 of the transport packaging 2 until the cavity 22 with the liquid 21 within is liquid-tight sealed at the first end 13 of the transport packaging 2, which is shown in figure 4.

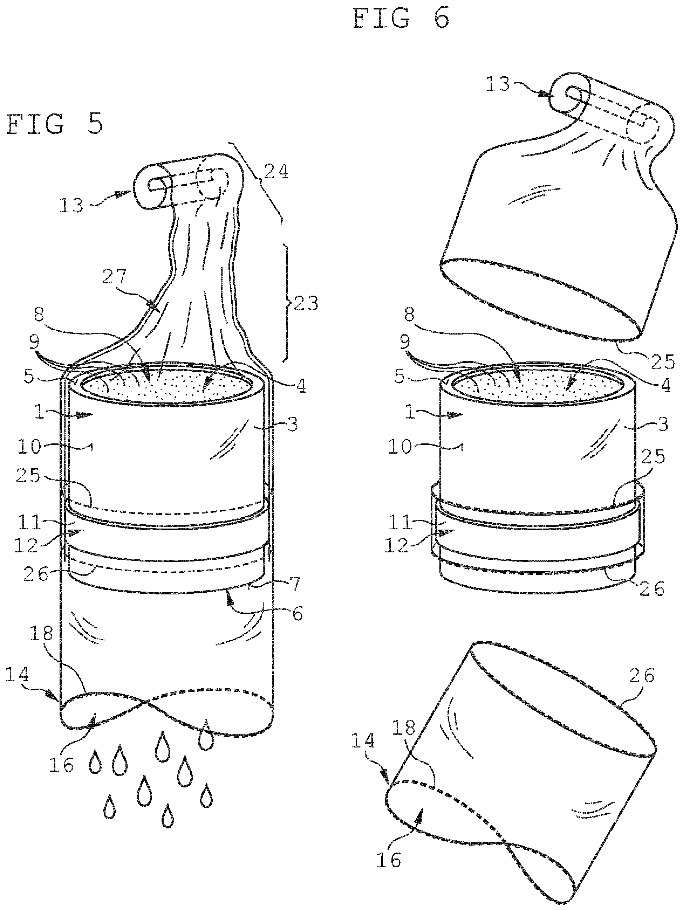

During a venting step shown in figure 5, the quantity of liquid 21 in the pouch 27, i.e. inside the closed cavity 22 is forced through the hollow fiber membrane filter 1 by compressing the cavity 22 and reducing the volume of the cavity 22. This can be done manually by either pressing together opposite sides of the cavity section 23, or by adding further winding movements to the wound-up first end 13 of the transport packaging 2. By reducing the volume of the cavity 22 a pressure is built up which forces the liquid 21 through the housing 3 of the hollow fiber membrane filter 1 and to exit at the second front side 7 of the housing 3 of the hollow fiber membrane filter 1. The liquid 21 that has passed through the housing 3 and the hollow fiber material therein exits the transport packaging 2 through the second opening 16 at the second end 14 of the transport packaging 2. Due to the pressure that is applied to the cavity 22 and the liquid 21 therein, the liquid 21 is forced through the housing 3 within a much shorter duration than a soaking process with only the gravitational force pushing the liquid 21 through the housing 3, resulting in a slow soaking process that requires much more time than the time that is required for applying pressure to the cavity and forcing the liquid 21 at a high flow rate through the housing 3. After most or all of the liquid 21 has been forced through the housing 3, the venting step is completed. If required, the transport packaging filling step and the venting step can be performed repeatedly in order to force a large amount of liquid 21 through the housing 3, whereby the large amount of liquid 21 is larger than the volume of the cavity 22.

After the full venting process has been completed, the first end 13 of the transport packaging 2 that protrudes over the housing 3 can be separated from the housing 3 by pulling the first end 13 away from the housing 3, resulting in severing a third perforation 25 next to the liquid-tight sealing 12 along the circumferential line of the housing **3.** Then the housing 3 of the hollow fiber membrane filter 1 can be inserted with the first front side 5 of the housing 3 first into a filtering device that is not shown in the figures. During the mounting of the housing 3 into the filtering device the hollow fiber membrane filter 1 can be handled by a user by only touching the second end 14 of the transport packaging 2 that still remains attached to the housing 3 during the insertion of the housing 3 into the filtering device. After fixing the housing 3 of the hollow fiber membrane filter 1 into the filtering device, the second end 14 of the transport packaging 2 can also be removed by pulling the second end 14 away from the housing 3. This will sever a fourth perforation 26 and allow the second end 14 to be separated from the housing 3 of the hollow fiber membrane filter 1.

It is also possible to begin with removing the second end 14 of the transport packaging 2 before removing the first end 13 of the transport packaging 2 afterwards. The sequence of the successive removals of the first and second end 13, 14 can be adapted to the manner in which the housing 3 is mounted into the filtering device.

By attaching such a transport packaging 2 in a liquid-tight manner to the housing 3 of the hollow fiber membrane filter **1,** the required venting process of the hollow fiber membrane filter 1 can be performed manually and without need for additional tools or elements within a very short time. Afterwards, the hollow fiber membrane filter 1 can be inserted into a filtering device without touching the housing 3 of the hollow fiber membrane filter, thus reducing the risk of any unwanted contamination of the hollow fiber membrane filter 1 during the venting process and the subsequent mounting into the filtering device. The transport packaging 2 can be manufactured in a very cost-effective manner and can be easily attached to the housing 3 by well-known methods like e.g. ultrasonic welding, providing at the same time for a liquid-tight sealing 12.

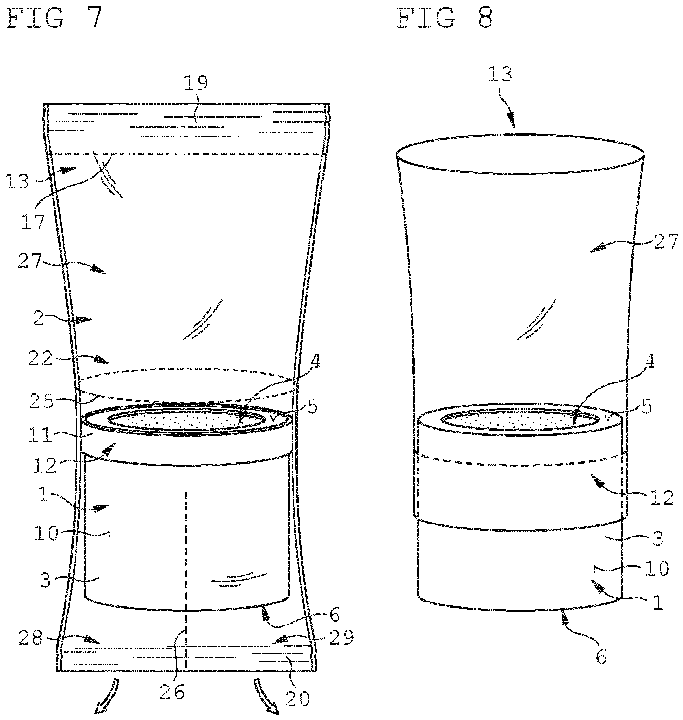

Figure 7 illustrates another embodiment of the hollow fiber membrane filter 1, whereby only the design of a part of the transport packaging 2 is different from the embodiment shown in Figures 1 to 6. The first end 13 with the first end part 19 and the cavity 22 of the transport packaging 2 is similar to the embodiment shown in Figures 1 to 6. However, the second end 14 and in particular the second opening 16 differs. Instead of a forth perforation 26 that runs around a circumferential line of the housing 3 next to the liquid-tight sealing 12, the forth perforation 26 runs perpendicular to the circumferential line and perpendicular to the alignment of the second end part 20 of the transport packaging 2. The forth perforation 26 begins at the second end part 20 and extends in the direction of the housing 3 with the liquid-tight sealing 12 that is located next to the first front side 5 of the housing 3. The forth perforation 26 can end at a distance to the liquid-tight sealing 12, as the forth perforation 26 is meant to allow for a forced opening of the transport packaging 2 and to provide for a forced separation of two separated parts 28, 29 at the second end part 20. This will enable a person to grasp both separated parts 28, 29 and to strip off and to remove the complete transport packaging 2 from the housing 3. In order to facilitate such a complete removal of the transport packaging 2 after completion of the venting step, the liquid-tight sealing 12 can be designed in a manner that allows for a residue-free removal of the ring of adhesive 11 from the housing 3.

Figure 8 illustrates another embodiment of the hollow fiber membrane filter 1 according to the invention. Instead of a transport packaging 2 that is affixed at the housing 3 with the liquid-tight sealing 12, there is only a separate pouch 27 affixed at the housing 3. The separate pouch 27 can be made of a flexible plastic film or an impregnated paper material which is impermeable to water for at least a few minutes. The pouch 27 is affixed by ultrasonic welding or heat-shrinking to the housing 3. Preferably, the pouch 27 is manufactured from a biodegradable or recycled material. The shape of the pouch 27 can be tubular with a constant cross-section area. The shape of the pouch 27 can also be conical or bellied to allow for a cross-section area that is larger than the cross-section area of the housing 3 of the hollow fiber membrane filter 1. The pouch 27 can be open-ended at the first end 13 that is opposite to the liquid-tight sealing 12. The pouch 27 can also be closed at the first end 13 in order to prevent any contamination of the inside of the pouch 27 before performing the venting step.

In addition to the embodiment shown in Figure 8, the hollow fiber membrane filter 1 and the pouch 27 can be arranged inside of a transport packaging that is not affixed to the hollow fiber membrane filter with a liquid-tight sealing. The transport packaging can be any transport packaging that is known from prior art, e.g. a cardboard packaging or a fabric bag. The pouch 27 does not require much space inside of the transport packaging.

## Claims

1. Method for venting a hollow fiber membrane filter (1) before first use of the filter in a filtering device,
whereby a liquid (21) is forced through the hollow fiber membrane filter (1),
wherein, during a pouch filling step, a quantity of a liquid (21) is filled into a cavity (22) formed by a flexible and liquid-tight open pouch (27) that is sealed liquid-tight along a circumferential surface of a housing (3) of the hollow fiber membrane filter (1) at a first front side (5) of the housing (3) of the hollow fiber membrane filter (1),
wherein, during the pouch filling step, after the filling of the liquid (21) into the cavity (22) through a first opening (15) at a first end (13) of the pouch (27), the first end (13) of the pouch (2) is closed in order to enclose the quantity of the liquid (21) in the pouch (27) at the first front side (4) of the housing (3) of the hollow fiber membrane filter (1),
wherein, during a venting step, the quantity of liquid (21) in the cavity (22) is forced through the hollow fiber membrane filter (1) to exit at the second front side (6) of the housing (3) of the hollow fiber membrane filter (1) by reducing the size of the cavity (22) formed by the closed first end (13) of the pouch (27), and
wherein, after the venting process has been performed and completed, the hollow fiber membrane filter is mounted into the filtering device.

2. Method according to claim 1, wherein, during the pouch filling step, after the filling of the liquid (21) into the cavity (22), the first opening (15) of the pouch (27) is closed by several winding movements of the first end (13) of the pouch (27) around a winding axis.

3. Method according to claim 1 or 2, wherein the size of the cavity (22) is reduced by manually compressing the flexible pouch (27).

4. Method according to any of the preceding claims, wherein, after venting the hollow fiber membrane filter (1), at least one part of the pouch (27) that protrudes over the housing (3) of the hollow fiber membrane filter (1) is separated and removed.

5. Method according to any of the preceding claims, wherein, during a pouch preparation step a flexible and liquid-tight closed transport packaging (2), that can be used as the pouch (27) and that is sealed liquid-tight along a circumferential surface of a housing (3) of the hollow fiber membrane filter (1), is opened at a first end (13) and opened at a second end (14) providing first and second openings (15, 16) of the transport packaging (2) with access to corresponding first and second openings (4, 6) of the hollow fiber membrane filter (1) at the opposite front sides (5, 7) of the housing (3) of the hollow fiber membrane filter (1).

6. Method according to claim **5,** wherein, during a transport packaging removal step, at least one part of the transport packaging (2) with the first end (13) that protrudes over the housing (3) of the hollow fiber membrane filter (1) and at least one part of the transport packaging (2) with the second end (14) is separated from the housing (3) and removed.

7. Hollow fiber membrane filter device for performing a method according to any one of the preceding claims, comprising a hollow fiber membrane filter (1) for mounting into a filtering device, the hollow membrane filter (1) comprising a housing (3) that encloses a hollow fiber material (8), wherein a flexible and liquid-tight open pouch (27) is sealed liquid-tight along a circumferential surface of the housing (3) of the hollow fiber membrane filter (1) at a first front side (5) of the housing (3) of the hollow fiber membrane filter (1).

8. Hollow fiber membrane filter device according to claim 7 and with a transport packaging (2) that encloses the housing (3) after manufacturing and before using the hollow fiber membrane filter (1),
wherein the transport packaging (2) is made of a flexible and liquid-tight transport packaging material, and
wherein the transport packaging (2) is sealed liquid-tight along a circumferential surface of the housing (3) of the hollow fiber membrane filter (1) with a first end (13) of the transport packaging (2) covering a first front side (5) of the housing (3) and with a second end (14) of the transport packaging (2) covering a second front side (7) of the housing (3), whereby the pouch (27) is formed by a cavity section between the first end part (19) of the transport packaging (2) and the first front side (5) of the housing (3) of the hollow fiber membrane filter (1).

9. Hollow fiber membrane filter device according to claim **8,** wherein a cross-section of the transport packaging (2) is adapted to a cross-section of the housing (3) along the circumferential surface of the housing (3) with the liquid-tight sealing (12).

10. Hollow fiber membrane filter device according to claim 9, wherein the housing (3) of the hollow fiber membrane filter (1) is tube-shaped and wherein the transport packaging (2) is tube-shaped.

11. Hollow fiber membrane filter device according to any of the preceding claims 8 to 10,
wherein a first perforation (17) is arranged at the first end (13) of the transport packaging (2) that allows for removal of a first end part (19) of the transport packaging (2) at a distance to the housing (3) of the hollow fiber membrane filter (1), resulting in a cavity (22) formed by a cavity section (23) of the first end (13) of the transport packaging (2) between the removed first end part (19) of the transport packaging (2) and the first front side (5) of the housing (3) of the hollow fiber membrane filter (1).

12. Hollow fiber membrane filter device according to claim 11, wherein the first end (13) of the transport packaging (2) also comprises a closure section (24) between the cavity section (23) and the first end part (19) of the transport packaging (2) that allows for closing the cavity section (23) after removal of the first end part (19) of the transport packaging (2).

13. Hollow fiber membrane filter device according to any of the preceding claims 8 to 12, wherein a second perforation (18) is arranged at the second end (14) of the transport packaging (2) that allows for removal of a second end part (20) of the transport packaging.

14. Hollow fiber membrane filter device according to any of the preceding claims 8 to 13, wherein a third perforation (25) is arranged at the first end (13) of the transport packaging (2) next to the fluid-tight sealing (12) of the transport packaging (2) along the circumferential surface of the housing (3) of the hollow fiber membrane filter (1).

15. Hollow fiber membrane filter device according to any of the preceding claims 8 to 14, wherein a fourth perforation (26) is arranged at the second end (14) of the transport packaging (2) next to the fluid-tight sealing (12) of the transport packaging (2) along the circumferential surface of the housing (3) of the hollow fiber membrane filter (1).

## Patentansprüche

1. Verfahren zum Entlüften eines Hohlfasermembranfilters (1) vor dem ersten Einsatz des Filters in einer Filtervorrichtung, wobei eine Flüssigkeit (21) durch den Hohlfasermembranfilter (1) gedrückt wird,
wobei während eines Beutelfüllschritts eine Menge einer Flüssigkeit (21) in einen Hohlraum (22) gefüllt wird, der durch einen flexiblen und flüssigkeitsdichten offenen Beutel (27) gebildet wird, der entlang einer Umfangsfläche eines Gehäuses (3) des Hohlfasermembranfilters (1) an einer ersten Vorderseite (5) des Gehäuses (3) des Hohlfasermembranfilters (1) flüssigkeitsdicht versiegelt ist,
wobei während des Beutelfüllschritts nach dem Füllen der Flüssigkeit (21) in den Hohlraum (22) durch eine erste Öffnung (15) an einem ersten Ende (13) des Beutels (27) das erste Ende (13) des Beutels (2) geschlossen wird, um die Menge der Flüssigkeit (21) in dem Beutel (27) an der ersten Vorderseite (4) des Gehäuses (3) des Hohlfasermembranfilters (1) zu umschließen,
wobei während eines Entlüftungsschritts die Menge an Flüssigkeit (21) in dem Hohlraum (22) durch den Hohlfasermembranfilter (1) gedrückt wird, um an der zweiten Vorderseite (6) des Gehäuses (3) des Hohlfasermembranfilters (1) auszutreten, indem die Größe des durch das geschlossene erste Ende (13) des Beutels (27) gebildeten Hohlraums (22) reduziert wird, und
wobei, nachdem der Entlüftungsprozess durchgeführt und abgeschlossen worden ist, der Hohlfasermembranfilter in die Filtervorrichtung montiert wird.

2. Verfahren nach Anspruch 1, wobei während des Beutelfüllschritts, nach dem Füllen der Flüssigkeit (21) in den Hohlraum (22), die erste Öffnung (15) des Beutels (27) durch mehrere Wickelbewegungen des ersten Endes (13) des Beutels (27) um eine Wickelachse geschlossen wird.

3. Verfahren nach Anspruch 1 oder 2, wobei die Größe des Hohlraums (22) durch manuelles Zusammendrücken des flexiblen Beutels (27) reduziert wird.

4. Verfahren nach einem der vorstehenden Ansprüche, wobei nach dem Entlüften des Hohlfasermembranfilters (1) mindestens ein Teil des Beutels (27), der über das Gehäuse (3) des Hohlfasermembranfilters (1) vorsteht, getrennt und entfernt wird.

5. Verfahren nach einem der vorstehenden Ansprüche, wobei in einem Beutelvorbereitungsschritt eine flexible und flüssigkeitsdicht verschlossene Transportverpackung (2), die als der Beutel (27) verwendet werden kann und die entlang einer Umfangsfläche eines Gehäuses (3) des Hohlfasermembranfilters (1) flüssigkeitsdicht versiegelt ist, an einem ersten Ende (13) geöffnet ist und an einem zweiten Ende (14) geöffnet ist, wodurch erste und zweite Öffnungen (15, 16) der Transportverpackung (2) mit Zugang zu entsprechenden ersten und zweiten Öffnungen (4, 6) des Hohlfasermembranfilters (1) an den gegenüberliegenden Vorderseiten (5, 7) des Gehäuses (3) des Hohlfasermembranfilters (1) bereitgestellt werden.

6. Verfahren nach Anspruch 5, wobei während eines Transportverpackungsentfernungsschritts mindestens ein Teil der Transportverpackung (2) mit dem ersten Ende (13), das über das Gehäuse (3) des Hohlfasermembranfilters (1) vorsteht, und mindestens ein Teil der Transportverpackung (2) mit dem zweiten Ende (14) von dem Gehäuse (3) getrennt und entfernt wird.

7. Hohlfasermembranfiltervorrichtung zum Durchführen eines Verfahrens nach einem der vorstehenden Ansprüche, umfassend einen Hohlfasermembranfilter (1) zum Montieren in eine Filtervorrichtung, wobei der Hohlfasermembranfilter (1) ein Gehäuse (3) umfasst, das ein Hohlfasermaterial (8) umschließt, wobei ein flexibler und flüssigkeitsdichter offener Beutel (27) entlang einer Umfangsfläche des Gehäuses (3) des Hohlfasermembranfilters (1) an einer ersten Vorderseite (5) des Gehäuses (3) des Hohlfasermembranfilters (1) flüssigkeitsdicht versiegelt ist.

8. Hohlfasermembranfiltervorrichtung nach Anspruch 7 und mit einer Transportverpackung (2), die das Gehäuse (3) nach Herstellung und vor Verwendung des Hohlfasermembranfilters (1) umschließt,
wobei die Transportverpackung (2) aus einem flexiblen und flüssigkeitsdichten Transportverpackungsmaterial hergestellt ist, und
wobei die Transportverpackung (2) entlang einer Umfangsfläche des Gehäuses (3) des Hohlfasermembranfilters (1) flüssigkeitsdicht versiegelt ist, wobei ein erstes Ende (13) der Transportverpackung (2) eine erste Vorderseite (5) des Gehäuses (3) abdeckt und wobei ein zweites Ende (14) der Transportverpackung (2) eine zweite Vorderseite (7) des Gehäuses (3) abdeckt, wobei der Beutel (27) durch einen Hohlraumabschnitt zwischen dem ersten Endteil (19) der Transportverpackung (2) und der ersten Vorderseite (5) des Gehäuses (3) des Hohlfasermembranfilters (1) gebildet wird.

9. Hohlfasermembranfiltervorrichtung nach Anspruch 8,
wobei ein Querschnitt der Transportverpackung (2) an einen Querschnitt des Gehäuses (3) entlang der Umfangsfläche des Gehäuses (3) mit der flüssigkeitsdichten Versiegelung (12) angepasst ist.

10. Hohlfasermembranfiltervorrichtung nach Anspruch 9,
wobei das Gehäuse (3) des Hohlfasermembranfilters (1) röhrenförmig ist und wobei die Transportverpackung (2) röhrenförmig ist.

11. Hohlfasermembranfiltervorrichtung nach einem der vorstehenden Ansprüche 8 bis 10,
wobei eine erste Perforation (17) an dem ersten Ende (13) der Transportverpackung (2) angeordnet ist, die ein Entfernen eines ersten Endteils (19) der Transportverpackung (2) in einem Abstand zum Gehäuse (3) des Hohlfasermembranfilters (1) ermöglicht, was zu einem Hohlraum (22) führt, der durch einen Hohlraumabschnitt (23) des ersten Endes (13) der Transportverpackung (2) zwischen dem entfernten ersten Endteil (19) der Transportverpackung (2) und der ersten Vorderseite (5) des Gehäuses (3) des Hohlfasermembranfilters (1) gebildet wird.

12. Hohlfasermembranfiltervorrichtung nach Anspruch 11, wobei das erste Ende (13) der Transportverpackung (2) auch einen Verschlussabschnitt (24) zwischen dem Hohlraumabschnitt (23) und dem ersten Endteil (19) der Transportverpackung (2) umfasst, der Schließen des Hohlraumabschnitts (23) nach Entfernen des ersten Endteils (19) der Transportverpackung (2) ermöglicht.

13. Hohlfasermembranfiltervorrichtung nach einem der vorstehenden Ansprüche 8 bis 12, wobei eine zweite Perforation (18) an dem zweiten Ende (14) der Transportverpackung (2) angeordnet ist, die Entfernen eines zweiten Endteils (20) der Transportverpackung ermöglicht.

14. Hohlfasermembranfiltervorrichtung nach einem der vorstehenden Ansprüche 8 bis 13, wobei eine dritte Perforation (25) an dem ersten Ende (13) der Transportverpackung (2) neben der fluiddichten Versiegelung (12) der Transportverpackung (2) entlang der Umfangsfläche des Gehäuses (3) des Hohlfasermembranfilters (1) angeordnet ist.

15. Hohlfasermembranfiltervorrichtung nach einem der vorstehenden Ansprüche 8 bis 14, wobei eine vierte Perforation (26) an dem zweiten Ende (14) der Transportverpackung (2) neben der fluiddichten Versiegelung (12) der Transportverpackung (2) entlang der Umfangsfläche des Gehäuses (3) des Hohlfasermembranfilters (1) angeordnet ist.

## Revendications

1. Procédé de ventilation d'un filtre à membrane à fibres creuses (1) avant la première utilisation du filtre dans un dispositif de filtrage, selon lequel un liquide (21) est forcé à travers le filtre à membrane à fibres creuses (1),
dans lequel, pendant une étape de remplissage de poche, une quantité d'un liquide (21) est introduite dans une cavité (22) formée par une poche ouverte (27), souple et étanche aux liquides qui est scellée de manière étanche aux liquides le long d'une surface circonférentielle d'un logement (3) du filtre à membrane à fibres creuses (1) au niveau d'un premier côté avant (5) du logement (3) du filtre à membrane à fibres creuses (1),
dans lequel, pendant l'étape de remplissage de poche, après l'introduction du liquide (21) dans la cavité (22) à travers une première ouverture (15) au niveau d'une première extrémité (13) de la poche (27), la première extrémité (13) de la poche (2) est fermée afin d'enfermer la quantité du liquide (21) dans la poche (27) au niveau du premier côté avant (4) du logement (3) du filtre à membrane à fibres creuses (1),
dans lequel, pendant une étape de ventilation, la quantité de liquide (21) dans la cavité (22) est forcée à travers le filtre à membrane à fibres creuses (1) pour sortir au niveau du second côté avant (6) du logement (3) du filtre à membrane à fibres creuses (1) en réduisant la taille de la cavité (22) formée par la première extrémité fermée (13) de la poche (27), et
dans lequel, après que le processus de ventilation a été effectué et terminé, le filtre à membrane à fibres creuses est monté dans le dispositif de filtrage.

2. Procédé selon la revendication 1, dans lequel, pendant l'étape de remplissage de poche, après l'introduction du liquide (21) dans la cavité (22), la première ouverture (15) de la poche (27) est fermée par plusieurs mouvements d'enroulement de la première extrémité (13) de la poche (27) autour d'un axe d'enroulement.

3. Procédé selon la revendication 1 ou 2, dans lequel la taille de la cavité (22) est réduite en comprimant manuellement la poche souple (27).

4. Procédé selon l'une quelconque des revendications précédentes, dans lequel, après la ventilation du filtre à membrane à fibres creuses (1), au moins une partie de la poche (27) qui fait saillie sur le logement (3) du filtre à membrane à fibres creuses (1) est séparée et retirée.

5. Procédé selon l'une quelconque des revendications précédentes, dans lequel, pendant une étape de préparation de poche, un emballage de transport fermé, souple et étanche aux liquides (2), qui peut être utilisé en tant que poche (27) et qui est scellé de manière étanche aux liquides le long d'une surface circonférentielle d'un logement (3) du filtre à membrane à fibres creuses (1), est ouvert au niveau d'une première extrémité (13) et ouvert au niveau d'une seconde extrémité (14) fournissant des première et seconde ouvertures (15, 16) de l'emballage de transport (2) avec accès aux première et seconde ouvertures (4, 6) correspondantes du filtre à membrane à fibres creuses (1) au niveau des côtés avant opposés (5, 7) du logement (3) du filtre à membrane à fibres creuses (1).

6. Procédé selon la revendication 5, dans lequel, pendant une étape de retrait d'emballage de transport, au moins une partie de l'emballage de transport (2) avec la première extrémité (13) qui fait saillie sur le logement (3) du filtre à membrane à fibres creuses (1) et au moins une partie de l'emballage de transport (2) avec la seconde extrémité (14) sont séparées du logement (3) et retirées.

7. Dispositif de filtre à membrane à fibres creuses pour la réalisation d'un procédé selon l'une quelconque des revendications précédentes, comprenant un filtre à membrane à fibres creuses (1) destiné à être monté dans un dispositif de filtrage, le filtre à membrane à fibres creuses (1) comprenant un logement (3) qui enferme un matériau à fibres creuses (8), dans lequel une poche ouverte, souple et étanche aux liquides (27) est scellée de manière étanche aux liquides le long d'une surface circonférentielle du logement (3) du filtre à membrane à fibres creuses (1) au niveau d'un premier côté avant (5) du logement (3) du filtre à membrane à fibres creuses (1).

8. Dispositif de filtre à membrane à fibres creuses selon la revendication 7 et doté d'un emballage de transport (2) qui enferme le logement (3) après la fabrication et avant l'utilisation du filtre à membrane à fibres creuses (1),
dans lequel l'emballage de transport (2) est constitué d'un matériau d'emballage de transport souple et étanche aux liquides, et
dans lequel l'emballage de transport (2) est scellé de manière étanche aux liquides le long d'une surface circonférentielle du logement (3) du filtre à membrane à fibres creuses (1) avec une première extrémité (13) de l'emballage de transport (2) couvrant un premier côté avant (5) du logement (3) et avec une seconde extrémité (14) de l'emballage de transport (2) couvrant un second côté avant (7) du logement (3), selon lequel la poche (27) est formée par une section de cavité entre la première partie d'extrémité (19) de l'emballage de transport (2) et le premier côté avant (5) du logement (3) du filtre à membrane à fibres creuses (1).

9. Dispositif de filtre à membrane à fibres creuses selon la revendication 8,
dans lequel une section transversale de l'emballage de transport (2) est adaptée à une section transversale du logement (3) le long de la surface circonférentielle du logement (3) avec le scellement étanche aux liquides (12).

10. Dispositif de filtre à membrane à fibres creuses selon la revendication 9,
dans lequel le logement (3) du filtre à membrane à fibres creuses (1) est en forme de tube et dans lequel l'emballage de transport (2) est en forme de tube.

11. Dispositif de filtre à membrane à fibres creuses selon l'une quelconque des revendications précédentes 8 à 10,
dans lequel une première perforation (17) est agencée au niveau de la première extrémité (13) de l'emballage de transport (2) qui permet le retrait d'une première partie d'extrémité (19) de l'emballage de transport (2) à une certaine distance du logement (3) du filtre à membrane à fibres creuses (1), résultant en une cavité (22) formée par une section de cavité (23) de la première extrémité (13) de l'emballage de transport (2) entre la première partie d'extrémité (19) retirée de l'emballage de transport (2) et le premier côté avant (5) du logement (3) du filtre à membrane à fibres creuses (1).

12. Dispositif de filtre à membrane à fibres creuses selon la revendication 11, dans lequel la première extrémité (13) de l'emballage de transport (2) comprend également une section de fermeture (24) entre la section de cavité (23) et la première partie d'extrémité (19) de l'emballage de transport (2) qui permet la fermeture de la section de cavité (23) après le retrait de la première partie d'extrémité (19) de l'emballage de transport (2).

13. Dispositif de filtre à membrane à fibres creuses selon l'une quelconque des revendications précédentes 8 à 12, dans lequel une deuxième perforation (18) est agencée au niveau de la seconde extrémité (14) de l'emballage de transport (2) qui permet le retrait d'une seconde partie d'extrémité (20) de l'emballage de transport.

14. Dispositif de filtre à membrane à fibres creuses selon l'une quelconque des revendications précédentes 8 à 13, dans lequel une troisième perforation (25) est agencée au niveau de la première extrémité (13) de l'emballage de transport (2) à côté du scellement étanche aux fluides (12) de l'emballage de transport (2) le long de la surface circonférentielle du logement (3) du filtre à membrane à fibres creuses (1).

15. Dispositif de filtre à membrane à fibres creuses selon l'une quelconque des revendications précédentes 8 à 14, dans lequel une quatrième perforation (26) est agencée au niveau de la seconde extrémité (14) de l'emballage de transport (2) à côté du scellement étanche aux fluides (12) de l'emballage de transport (2) le long de la surface circonférentielle du logement (3) du filtre à membrane à fibres creuses (1).
